# EUROPÄISCHE PATENTSCHRIFT

(11) **EP 1 820 610 B1**
(45) Veröffentlichungstag und Bekanntmachung des Hinweises auf die Patenterteilung: **22.04.2009**
(21) Anmeldenummer: 07101964.0
(22) Anmeldetag: 08.02.2007
(51) Int. Cl.: B25J 9/10, B25J 15/00, F16H 19/00, F16H 57/12, A61F 2/58

(54) **Roboterhand**
Robot hand
Main de robot

(30) Priorität: 11.02.2006 DE 102006006322
(43) Veröffentlichungstag der Anmeldung: 22.08.2007
(73) Patentinhaber: Deutsches Zentrum für Luft- und Raumfahrt e.V., 51147 Köln (DE)
(72) Erfinder: Grebenstein, Markus, 80799 München (DE)
(74) Vertreter: von Kirschbaum, Alexander

(56) Entgegenhaltungen:
- FR-A1- 2 832 345
- JP-A- 3 196 983
- JP-A- 6 006 953
- JP-A- 6 008 178
- JP-A- 2004 306 224
- US-A- 4 865 376
- US-A- 4 946 380
- US-A- 5 046 375
- US-A- 5 502 363
- US-A- 5 762 390
- US-B1- 6 668 678

## Beschreibung

Die Erfindung betrifft eine Roboterhand.

Roboterhände weisen häufig entsprechend einer menschlichen Hand mehrere Finger mit mindestens zwei, häufig drei, Fingerelementen, bzw. Fingergliedern, auf. Ebenfalls in Nachempfindung der menschlichen Hand sind die Finger mit einem Handtellerelement verbunden. Zur Bewegung der Finger, bzw. der Fingerelemente, sind zwischen den einzelnen Elementen Gelenke vorgesehen. Zum Betätigen der Elemente sind entweder Teile des Gelenks oder das Fingerelement mit einer Antriebseinrichtung verbunden, Die Antriebseinrichtungen selbst sind zumeist außerhalb der Roboterhand angeordnet, da die Roboterhand selbst zu filigran ist um hier auch Antriebseinrichtungen vorzusehen. Die Verbindung zwischen der Antriebseinrichtung und den einzelnen Fingerelementen bzw. Gelenken erfolgt z. B. über Stangenhebel und/oder Bowdenzüge. Die Antriebseinrichtungen können im Unterarm, in einer externen Antriebsbox oder auch im Bereich des Handtellerelements angeordnet sein.

Um mit einer Roboterhand exakt greifen zu können und möglichst exakte Handhabungen durchführen zu können, weisen die einzelnen Elemente der Roboterhand eine möglichst hohe Steifigkeit auf. Dies hat allerdings den Nachteil, dass bei gewollten oder ungewollten Kollisionen hohe Impulse auf die einzelnen Elemente wirken und in die Antriebseinrichtung übertragen werden.

Dies kann zur Beschädigung der Antriebseinrichtung, der Fingerelemente, etc. führen. Dies ist insbesondere bei Roboterhänden nachteilig, da die einzelnen Bauteile der Roboterhände sehr fragil sind und auf Grund des beschränkten Raums nicht stabil oder massiv ausgebildet werden können.

Um durch Kollisionen hervorgerufene Beschädigungen zu vermeiden, ist ein aktives Ausweichen bzw. eine entsprechende Antriebsregelung bei Kollisionen bekannt. Dies ist zwar prinzipiell möglich, weist jedoch neben einem beträchtlichen Steuerungsaufwand den Nachteil auf, dass das Auftreten einer Kollision, sowie deren Richtung und eventuell Stärke, bekannt sein muss. Die entsprechenden Daten müssen über Sensoren eingelesen und verarbeitet werden. Ein unmittelbares Reagieren bei einer unvorhergesehenen Kollision ist auf Grund der Trägheit des Systems, insbesondere des Antriebs, und auf Grund der Totzeiten zwischen dem Feststellen einer Kollision, der Datenaufnahme mittels Sensoren und dem Berechnen einer Reaktion, nicht möglich. Auch bei leistungsfähigen Robotern liegen derartige Totzeiten im Bereich von 2 bis 10 ms. Ferner weisen aktive Kollisionssysteme den Nachteil auf, dass bei ausgeschaltetem System keine Reaktion erfolgen kann.

Ein Reduzieren der Steifigkeit der Gelenke und Glieder der Roboterhand ist insbesondere für die Bewerkstelligung komplexer Greif- und Manipulationsaufgaben nicht möglich, da hierbei eine ausreichende Positionsgenauigkeit nicht mehr erreicht werden kann.

Roboterhände mit mehreren Fingerelementen, die mit einem Handelement verbunden sind, sind beispielsweise aus US 5,762,390 und US 4,946,308 bekannt. Zwischen den Fingerelementen, sowie zwischen den Handtellerelementen und dem ersten Fingerelement ist jeweils ein Gelenk angeordnet. Mit den Fingerelementen und/oder den Gelenken sind Antriebseinrichtungen zum Betätigen der Fingerelemente verbunden.

Aus US-A-6668678 ist eine Roboterhand gemäß dem Oberbegriff der Anspruchs 1 bekannt gewerden.

Aus US 5,502,363 ist ein Robotermanipulator bekannt, der nach dem antagonistischen Prinzip betätigt wird. Hierbei sind die Fingerelemente und/oder die Gelenke mit Antriebseinrichtungen verbunden, wobei mindestens eine der Antriebseinrichtungen mit einem Steifigkeits-Einstellelement zur Einstellung einer passiven Steifigkeit des Gelenks verbunden ist.

Aufgabe der Erfindung ist es eine Roboterhand zu schaffen, bei der die Gefahr von Beschädigungen bei gewollten oder ungewollten Kollisionen verringet ist.

Die Lösung der Aufgabe erfolgt erfindungsgemäß durch die Merkmale des Anspruchs 1.

Erfindungsgemäß weist die Antriebseinrichtung, die mit einem Gelenk und/oder einem Fingerelement verbunden ist, ein oder mehrer Steifigkeits-Einstellelemente auf. Mit Hilfe des Steifigkeits-Einstellelement ist es möglich, eine passive Steifigkeit eines Gelenks einzustellen. Dies hat den Vorteil, dass die Steifigkeit des Gelenks in Abhängigkeit der Situation, in der sich die Roboterhand befindet, eingestellt werden kann. Besteht beispielsweise auf Grund des Bewegens des Roboters oder des Bewegens andere Elemente in der Nähe des Roboters die Gefahr von Kollisionen, so kann die Steifigkeit, mit Hilfe des erfindungsgemäßen Steifigkeits-Einstellelements, sehr nachgiebig eingestellt werden, so dass die Gefahr von Beschädigungen bei einem tatsächlichen Auftreten einer Kollision deutlich verringert ist. Andererseits ist es auf Grund des Vorsehens eines einstellbaren Steifigkeits-Einstellelements möglich, bei hochpräzisen Greif- und Manipulationsaufgaben eine hohe Steifigkeit einzustellen, um eine möglichst große Positionsgenauigkeit zu erzielen.

Bei der Erfindung ist nun eine Antriebseinrichtung stationär und die andere verschiebbar ausgebildet. Hierbei wird unter stationär verstanden, dass die Antriebseinrichtung gegenüber der Roboterhand stationär ist. Insbesondere mit Hilfe der verschiebbar angeordneten bzw. gelagerten Antriebseinrichtung, wird das Gelenk oder das Fingerelement betätigt. Diese Betätigung erfolgt, wie auch bei anderen Ausführungsformen, vorzugsweise unmittelbar durch eine Verbindung zwischen der Antriebseinrichtung und dem Gelenk und/oder über Zwischenteile wie Stangen, Hebel und/oder Bowdenzüge.

Beim diesen erfindungsgemäßen Vorsehen von zwei Antriebseinrichtungen je Gelenk, dessen Steifigkeit einstellbar sein soll, ist eine Antriebseinrichtung zur Einstellung der Steifigkeit, die andere Antriebseinrichtung zum Bewegen des Fingerelements vorgesehen. Hierbei ist vorzugsweise die Antriebseinrichtung, die zum Einstellen der Steifigkeit dient, stationär, und die andere Antriebseinrichtung verschiebbar angeordnet.

Da mit Hilfe des erfindungsgemäßen Steifigkeits-Einstellelements eine passive Steifigkeit eines Gelenks eingestellt werden kann, ist kein zusätzliches aktives Regeln erforderlich. Eine Totzeit, in der eine Datenaufnahme mit Hilfe von Sensoren und der anschließenden Verarbeitung erfolgt, ist hierbei nicht gegeben. Der Finger der Roboterhand gibt somit unmittelbar bei Auftreten einer Kollision nach. Die bereits während der Totzeit auftretenden Beschädigungen sind somit vermieden.

Ferner ist auch eine Überlagerung von aktivem und passivem Ausweichen möglich.

Neben der Möglichkeit mit Hilfe des Steifigkeits-Einstellelements unterschiedliche Steifigkeiten, je nach Situation in der sich die Roboterhand befindet, einzustellen, ist es auch möglich, beispielsweise während eines Greifvorgangs die Steifigkeit zu verändern.

Neben der Möglichkeit mit Hilfe des Steifigkeits-Einstellelements unterschiedliche Steifigkeiten, je nach Situation in der sich die Roboterhand befindet, einzustellen, ist es auch möglich, beispielsweise während eines Greifvorgangs die Steifigkeit zu verändern.

Das Steifigkeits-Einstellelement kann beispielsweise eine Flüssigkeit aufweisen, deren Kompressibilität veränderbar ist. Hierzu sind beispielsweise elektrorheologische Flüssigkeiten geeignet. Ebenso kann die Steifigkeit eines Systems dadurch eingestellt werden, dass die Flüssigkeit durch eine Öffnung mit veränderbarem Querschnitt gedrückt wird. Hierbei ist die Steifigkeit des Systems durch Verändern der Querschnittsfläche einer oder mehrerer Durchschnittsöffnungen einstellbar.

Insbesondere oder in Kombination mit geeigneten Flüssigkeitssystemen weist das Steifigkeits-Einstellelement vorzugsweise ein Federelement auf. Bei dem Federelement kann es sich um eine Spiralfeder, eine Schraubenfeder oder einen Elastomerkörper, insbesondere einen Elastomerkegel oder eine Elastomerkugel handeln. Selbstverständlich sind auch Kombinationen derartiger Federelemente möglich. Hierbei können die einzelnen Federelemente zueinander parallel und/oder in Serie angeordnet sein. Ein Variieren der Steifigkeit ist hierbei durch einzelnes Zu- oder Abschalten von einzelnen Federelementen möglich. Bei beispielsweise parallel geschalteten Federelementen kann das Zuschalten durch Aktivieren von Verbindungselementen, d. h. durch Herstellen oder Lösen einer Verbindung, erfolgen.

Vorzugsweise erfolgt das Einstellen des Steifigkeits-Elements durch ein Verändern der Länge der Steifigkeits-Einstellelemente in deren Längsrichtung, d. h. durch Komprimieren oder Dehnen der Einstellelemente. Hierbei erfolgt die Längenänderung vorzugsweise in Kraftübertragungsrichtung. Bei Einstellelementen aus Elastomer kann die Steifigkeit beispielsweise auch durch das Element umgebende Ringe, Anlageflächen und dergleichen erfolgen. Beispielsweise wird beim Komprimieren eines zylindrischen Steifigkeits-Einstellelement aus einem elastomeren Material der Außenumfang vergrößert. Durch Vorsehen eines den Zylinder umgebenden Rings aus einem Material mit einer geringeren Elastizität, kann die Steifigkeit eines derartigen Elements eingestellt werden. Insbesondere ist es möglich, mehrere, das Element vollständig oder teilweise umgebende, Ringe vorzusehen, wobei die Steifigkeit eines derartigen Steifigkeits-Einstellelements je nach Anzahl der das Element umgebenden Ringe variiert werden kann.

Bei einer besonders bevorzugten Ausführungsform ist zumindest eines der Federelemente derart ausgebildet, dass es eine nicht-lineare Kennlinie aufweist. Die Kraft ändert sich somit in Abhängigkeit des Weges nicht linear. Ebenso ist es möglich, dass das Steifigkeits-Einstellelement mehrere Federelemente aufweist, deren Kombination zu einer nicht-linearen Kennlinie führt.

Zur Längenveränderung des Steifigkeits-Einstellelement und somit zur Variierung der Steifigkeit ist vorzugsweise eine Einstelleinrichtung, bzw. eine Antriebseinrichtung wie ein Elektromotor vorgesehen. Hierbei kann durch den Elektromotor die Länge des Steifigkeits-Einstellelements variiert werden,

Bei einer besonders bevorzugten Ausführungsform dient die Antriebsseinrichtung nicht nur zum Einstellen der Steifigkeit des Steifigkeits-Einstellelements, sondern zumindest teilweise auch zum Bewegen der Gelenke bzw. der Fingerelemente. Die Einstelleinrichtung ist somit zusätzlich auch Bestandteil der Antriebseinrichtung.

Erfindungsgemäß sind zwei Antriebseinrichtungen vorgesehen, die jeweils über ein Übertragungselement mit dem Gelenk und/oder dem Fingerelement verbunden sind. Die beiden Antriebseinrichtungen dienen hierbei auch zum Schwenken des Gelenks bzw. des Fingerelements. Ferner ist vorzugsweise mit jedem Übertragungselement ein Federelement verbunden, wobei vorzugsweise das Federelement ein Teil des Übertragungselementes ist. Hierbei ist beispielsweise ein erster Teil des Übertragungselements mit der einen Seite und ein zweiter Teil des Übertragungselements mit der anderen Seite des Federelements verbunden.

Nachfolgend wird die Erfindung anhand bevorzugter Ausführungsformen unter Bezugnahme auf die anliegenden Zeichnungen näher erläutert.

Es zeigen:
- Fig. 1: eine schematische Prinzipskizze eines Gelenks einer Roboterhand mit einstellbarer, passiver Steifigkeit (antagonistischer Antrieb),
- Fig. 2: eine schematische Prinzipskizze einer ersten Ausführungsform eines Gelenks einer Roboterhand mit einstellbarer, passiver Steifigkeit, und
- Fig. 3: eine schematische Prinzipskizze einer zweiten Ausführungsform eines Gelenks einer Roboterhand mit einstellbarer, passiver Steifigkeit.

Bei den in den Figuren dargestellten Prinzipskizzen ist jeweils prinzipiell eine Ausführungsmöglichkeit eines Gelenks für eine Roboterhand mit einstellbarer, passiver Steifigkeit dargestellt. Es handelt sich hierbei um stark vereinfachte Prinzipdarstellungen, aus denen die prinzipielle Funktion einer bevorzugten Ausführungsform hervorgeht. In den Ausführungsbeispielen sind die selben oder ähnliche Bauteile mit den selben Bezugszeichen bezeichnet.

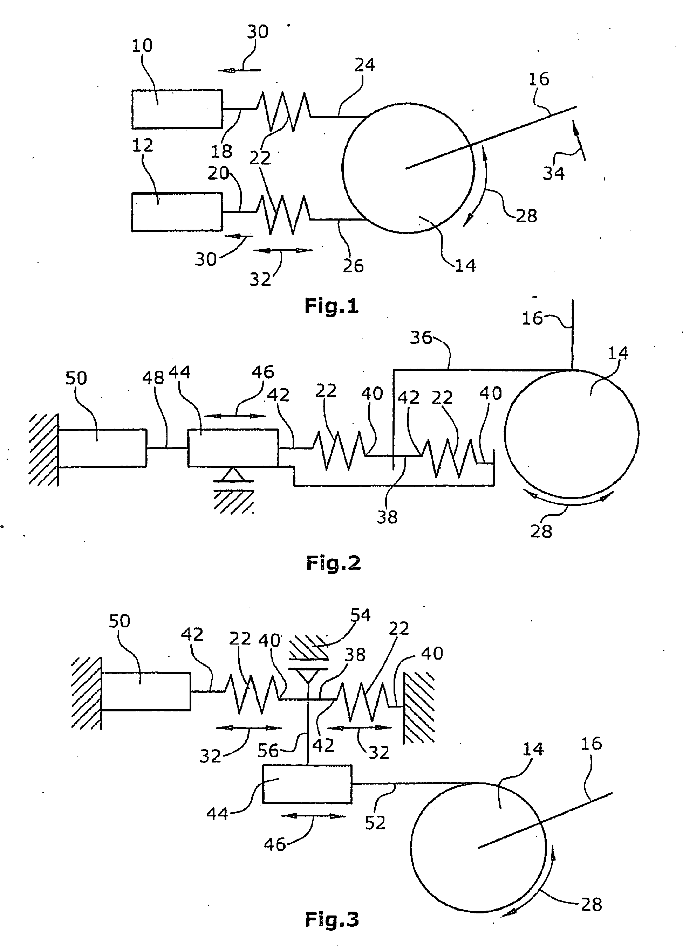

In der Prinzipskizze (Fig. 1) die nicht unter den Schutzbereich des Anspruchs 1 fällt, sind zwei Antriebseinrichtungen 10, 12 in Form von Elektromotoren vorgesehen. Die Antriebseinrichtung 10, 12 dienen ferner auch zum Betätigen eines Gelenks 14, bzw. eines mit dem Gelenk 14 verbundenen Fingerelements 16. Mit den beiden Motoren 10, 12 ist jeweils ein Übertragungselement 18 bzw. 20 verbunden. Bei den Übertragungselementen 18, 20 kann es sich beispielsweise um Seilzüge handeln. Die Übertragungselemente 18, 20 sind sodann jeweils mit einem Steifigkeits-Einstellelement 22 in Form einer Spiral- oder Schraubenfeder mit einer nicht-linearen Kennlinie verbunden. Bei dem Steifigkeits-Einstellelement handelt es sich in besonders bevorzugter Ausführungsform um eine aus geeignetem Material, insbesondere Elastomer, hergestellte Gummikugel. Auf der jeweils von dem Motor 10, 12 abgewandte Seite der Federelemente 22 sind weitere Übertragungselemente 24, 26 vorgesehen, die mit dem Gelenk 14 verbunden sind. Durch gegenläufiges Ansteuern der Motoren 10, 12 ist ein Schwenken des Gelenks 14 in Richtung eines Pfeils 28 möglich. Durch gleichförmiges Betätigen der Motoren 10,12 ist beispielsweise ein Ziehen an den Übertragungselementen 18, 20 jeweils in der Anspruchs 1 fällt, Richtung eines Pfeils 30 möglich. Hierdurch wird die Länge der Steifigkeits-Einstellelemente 22 in Kraftübertragungsrichtung 32 verändert und somit die Steifigkeit variiert.

Anstelle des Gelenks 14 kann auch ein Zwischenelement, wie eine Rolle, vorgesehen sein, an der entsprechend einer menschlichen Sehne ein Seilzug befestigt ist. Durch Betätigen der Motoren 10, 12 erfolgt beispielsweise ein Aufwickeln eines Seilzugs. Da dieser entsprechend einer menschlichen Sehne mit einem Fingerelement verbunden ist, kann hierdurch ggf. das Schwenken eines Fingerelements hervorgerufen werden.

Erfolgt durch eine auftretende Kollision eine Krafteinwirkung oder ein Impuls in Richtung eines Pfeils 34 auf das Fingerelement 16, kann dieser Impuls je nach eingestellter Steifigkeit der Steifigkeits-Einstellelemente 22 abgefangen werden. Eine Beschädigung einzelner Bauteile der Roboterhand, insbesondere des Antriebs, ist somit vermieden.

Bei einer ersten Ausführungsform (Fig. 2) ist das Fingerelement 16 ebenfalls mit einem Gelenk 14 verbunden. Das Gelenk 14 ist mit einem insbesondere steifen Übertragungselement 36 verbunden. Das Übertragungselement 36 ist über ein Zwischen- oder Übertragungselement 38 mit den beiden Federelementen 22 verbunden. Hierbei ist das Zwischenelement zwischen den beiden Federelementen 22 verbunden. Das Zwischenelement ist zwischen den beiden Federelementen 22 angeordnet, so dass die Verbindung zwischen Zwischenelement 38 und Übertragungselement 36 zwischen den beiden Federelementen 22 erfolgt. Die beiden Federelemente 22 weisen wiederum eine nicht-lineare Kennlinie auf. Das in Fig. 2 rechte Federelement 22 ist an einer Seite 40 fixiert. Die andere Seite 42 ist mit dem Zwischenelement 38 verbunden. Entsprechend ist das zweite Federelement 22 an der ersten Seite 40 mit dem Zwischenelement 38 und an der zweiten Seite 42 mit einer Antriebseinrichtung, wie einem Motor 44, verbunden. Der Motor 44 ist in Richtung eines Pfeils 46, d. h. in Längsrichtung der Steifigkeits-Einstellelemente 22 verschiebbar gelagert. Über ein Verbindungselement 48 ist der Motor 44 mit einem stationären Motor 50 verbunden. Durch Betätigen des stationären Motors 50 kann ein Verschieben des Motors in Richtung eines Pfeils 46 erfolgen. Mit Hilfe des Motors 44 wird die Steifigkeit durch Längung/Kürzung des zweiten Federelementes 22 verstellt. Das Schwenken des Gelenks 14 erfolgt durch den Motor 50, wobei hierbei sowohl der Motor 44 als auch das zweite Federelement 22 mitbewegt wird.

Bei einer weiteren bevorzugten Ausführungsform (Fig. 3) ist das Fingerelement 16 wiederum mit einem Gelenk 14 verbunden. Über ein Übertragungselement 52 ist das Gelenk 14 mit einem Motor 44 verbunden. Durch Betätigen des Motors 44 erfolgt ein Bewegen des Fingerelements 16 in Richtung des Pfeils 28. Der Motor 44 selber ist in Richtung eines Pfeils 46 über ein Lager 54 verschiebbar gelagert. Ferner ist der Motor 44 über ein Zwischenelement 56 mit einem Zwischenelement 38 verbunden. Das Zwischenelement 38 ist mit den beiden Federelementen 22 verbunden, wobei das in Fig. 3 rechte Ende 40 des Federelements 22 ortsfest ist, d. h. mit einem Teil der Roboterhand verbunden ist. Das rechte Ende 40 des Federelements 22 kann auch verschiebbar gelagert sein. Entsprechend dem in Fig. 2 dargestellten Ausführungsbeispiel ist auch der Motor 50 ortsfest angeordnet. Der Motor 50 ist gemäß dem dritten Ausführungsbeispiel mit einem Ende 42 der in Fig. 3 linken Feder verbunden.

Durch Betätigen des Motors 50 erfolgt ein Verändern der Länge der Federelemente 22 in Kraftübertragungsrichtung 32 und somit ein Einstellen der Steifigkeit.

## Patentansprüche

1. Roboterhand mit
mehreren, mindestens zwei Fingerelemente (16) aufweisenden Fingern,
einem mit den Fingern verbundenen Handtellerelement,
zwischen den Fingerelementen (16) sowie zwischen dem Handtellerelement und den ersten Fingerelementen (16) angeordneten Gelenken (14), und
mit den Fingerelementen (16) und/oder den Gelenken (14) verbundenen Antriebseinrichtungen (44, 50),
wobei mindestens eine Antriebseinrichtung (44, 50) mit einem Steifigkeits-Einstellelement (22,50,44) zum Einstellen einer passiven Steifigkeit des Gelenks (14) verbunden ist,
**dadurch gekennzeichnet, dass**
zwei Antriebseinrichtungen (44, 50) vorgesehen sind, wobei eine Antriebseinrichtung (50) stationär und die andere Antriebseinrichtung (44) verschiebbar ist, wobei eine Antriebseinrichtung (44,50) zur Steifigkeitseinstellung und die andere Antriebseinrichtung (50,44) zur Bewegung des Fingerelements (16) vorgesehen ist.

2. Roboterhand nach Anspruch 1, **dadurch gekennzeichnet, dass** die Steifigkeit des Steifigkeits-Einstellelements (22,44,50) durch eine Längenveränderung, insbesondere in Kraftübertragungsrichtung (32) erfolgt.

3. Roboterhand nach einem der Ansprüche 1 oder 2, **dadurch gekennzeichnet, dass** das Steifigkeits-Einstellelement (22,44,50) mit einer Antriebseinrichtung (50,44), insbesondere zur Längenveränderung, verbunden ist.

4. Roboterhand nach Anspruch 3, **dadurch gekennzeichnet, dass** die Antriebseinrichtung (50,44) zur Bewegung des Fingerelementes zusätzlich zum Betätigen der Gelenke (14) dient.

5. Roboterhand nach einem der Ansprüche 1 bis 4, **dadurch gekennzeichnet, dass** zwei Steifigkeits-Einstellelemente (22) hintereinander angeordnet sind.

6. Roboterhand nach Anspruch 5, **dadurch gekennzeichnet, dass** zwischen den zwei Steifigkeits-Einstellelementen (22) ein mit dem Gelenk (14) oder dem Fingerelement (16) verbundenes Übertragungselement (36) angeordnet ist.

7. Roboterhand nach einem der Ansprüche 5 oder 6, **dadurch gekennzeichnet, dass** eine Antriebseinrichtung (44) der beiden Antriebseinrichtungen (44,50) zwischen den beiden Steifigkeits-Einstellelementen (22) angeordnet ist.

## Claims

1. A robot hand comprising
a plurality of fingers having at least two finger elements (16) each,
a palm element connected with the fingers,
joints (14) arranged between said finger elements (16) as well as between said palm element and the first finger elements (16), and
drive means (44, 50) connected with said finger elements (16) and/or the joints (14),
at least one drive means (44, 50) being connected with a rigidity adjustment element (22, 50, 44) for adjusting the passive rigidity of the joint (14),
**characterized in that**
two drive means (44, 50) are provided, one drive means (50) being stationary and the other drive means (44) being displaceable, wherein one drive means (44, 50) is provided for rigidity adjustment and the other drive means (50, 44) is provided for moving said finger element (16).

2. The robot hand of claim 1, **characterized in that** the rigidity of the rigidity adjustment element (22, 44, 50) is obtained by a change in length, especially in the direction of force transmission (32).

3. The robot hand of one of claims 1 or 2, **characterized in that** the rigidity adjustment element (22, 44, 50) is connected with a drive means (44. 50), especially for changing the length.

4. The robot hand of claim 3, **characterized in that** the drive means (50, 44) for moving the finger element (16) additionally serves to actuate the joints (14).

5. The robot hand of one of claims 1 to 4, **characterized in that** two rigidity adjustment elements (22) are arranged one behind the other.

6. The robot hand of claim 5, **characterized in that** a transmission element (36) connected with the hinge (14) or the finger element (16) is provided between the two rigidity adjustment elements (22).

7. The robot hand of one of claims 5 or 6, **characterized in that** on drive means (44) of the two drive means (44, 50) is arranged between the two rigidity adjustment elements (22).

## Revendications

1. Main de robot comprenant
plusieurs doigts avec au moins deux éléments de doigt (16),
un élément de paume relié aux doigts,
des joints (14) disposés entre les éléments de doigt (16) aussi qu'entre l'élément de paume et les premiers éléments de doigt (16), et
des moyens d'entraînement (44, 50) reliés audits éléments de doigt (16) et/ou audits joints (14),
au moins un moyen d'entraînement (44, 50) étant relié à un élément d'ajustage de rigidité (22, 50, 44) pour ajuster la rigidité passive du joint (14),
**caractérisée en ce que**
deux moyens d'entraînement (44, 50) sont prévus, l'un des moyens d'entraînement (50) étant stationnaire et l'autre moyen d'entraînement (44) étant déplaçable, et un des moyens d'entraînement (44, 50) étant prévu pour l'ajustage de la rigidité tandis que l'autre moyen d'entraînement (50, 44) est prévu pour mouvoir l'élément de doigt (16).

2. Main de robot selon la revendication 1, **caractérisée en ce que** la rigidité de l'élément d'ajustage de rigidité (22, 44, 50) est ajustée par un changement en longueur, en particulier dans la direction) de transmission de force (32).

3. Main de robot selon une des revendications 1 ou 2, **caractérisée en ce que** l'élément d'ajustage de rigidité (22, 44, 50)est relié à un moyen d'entraînement (50, 44), en particulier pour le changement de la longueur.

4. Main de robot selon la revendication 3, **caractérisée en ce que** le moyen d'entraînement (50, 44) pour mouvoir de l'élément de doigt sert en outre à opérer les joints (14).

5. Main de robot selon une des revendications 1 à 4, **caractérisée en ce que** deux éléments d'ajustage de rigidité (22) sont disposés l'un derrière l'autre.

6. Main de robot selon la revendication 5, **caractérisée en ce qu'**un élément de transmission (36), relié audit joint (14) ou audit élément de doigt (16), est disposé entre les deux moyens d'ajustage de rigidité (22).

7. Main de robot selon une des revendications 5 ou 6, **caractérisée en ce que** l'un (44) des deux moyens d'entraînement (44, 50) est disposé entre les deux éléments d'ajustage de rigidité (22).
